# EUROPEAN PATENT SPECIFICATION

(11) **EP 0 425 538 B1**
(45) Date of publication and mention of the grant of the patent: **17.08.1994**
(21) Application number: 89908042.8
(22) Date of filing: 22.06.1989
(51) Int. Cl.: C07C 19/08, C07C 17/20

(54) **TaX5 CATALYZED HYDROFLUORINATION OF HALOGENATED ALKANES**
VERFAHREN ZUR HYDROFLUORIERUNG VON ALKANEN MIT HILFE DER TaX5-KATALYSATOREN
HYDROFLUORATION D'ALCANES CATALYSEE PAR TaX5

(30) Priority: 23.06.1988 US 210556
(43) Date of publication of application: 08.05.1991
(73) Proprietor: E.I. DU PONT DE NEMOURS AND COMPANY, Wilmington Delaware 19898 (US)
(72) Inventor: RAO, V., N., Mallikarjuna, Wilmington, DE 19809 (US)
(74) Representative: Woodcraft, David Charles
(86) International application number: US8902670
(87) International publication number: WO8912616

(56) References cited:
- US-A- 2 005 707
- US-A- 4 258 225

## Description

Process for the preparation of fluorinated alkanes by contacting halogenated alkanes with hydrogen fluoride in the presence of TaCl₅ or TaBr₅.

### BACKGROUND OF INVENTION

JP 52/103392 discloses and claims a catalyst for fluorinating a halogenated hydrocarbon which is obtained by mixing tantalum (V) halide with an antimony halide catalyst which consists of antimony (V) halide or a mixture consisting of said antimony halide and antimony (III) halide. If the resulting fluorination catalyst is used, the fluorination of a halogenated hydrocarbon can be accelerated more effectively than in a case when an unmodified antimony halide catalyst is used, and the production rate of the objective fluorinated compound per unit catalyst (weight) can be maximized. The patent clearly shows in Comparative Example 2 that tantalum pentachloride alone is ineffective as a fluorine exchange catalyst for the conversion of 1,1,1,2-tetrachlorodifluoroethane to 1,1,2-trichlorotrifluoroethane.

The need for environmentally suitable fluorocarbons for use as refrigerants, blowing agents and solvents has grown and spurred investigation of economically attractive routes to their production. The fluorinated alkanes produced by the process of this invention are useful as refrigerants, blowing agents or solvents per se or can be utilized as intermediates for the production of other halogenated alkanes which fill the same needs.

### SUMMARY OF THE INVENTION

This invention is a process for the preparation of fluorinated alkanes by contacting at a temperature of O°C to 185°C under substantially anhydrous conditions, one molar equivalent of a starting material selected from halogenated alkanes of the following formulas

R¹R²R³R⁴C and R⁵R⁶R⁷R⁸C

wherein
R¹, R², R³, R⁴, are CₓZ₂ₓ+1, wherein Z is H, F, Br, or Cl and x = 0 to 10 with the proviso that at least one of R¹, R², R³, or R⁴ be Cl or Br or contain Cl or Br;
R⁵ and R⁶ taken together are -(CH₂)ₙ wherein n is an integer from 2 to 7, wherein R⁷ and R⁸ are CₓZ₂ₓ+1, wherein Z is H, F, Br, or Cl and x = 0 to 10, with the proviso that at least R⁷ or R⁸ is Cl or Br or contains Cl or Br, with HF in the presence of at least one catalyst selected from tantalum pentachloride and tantalum pentabromide to produce reaction products; removing the reaction products from contact with the catalyst and isolating a fluorinated alkane having one or more fluorine atoms above the number present in the starting material.

### DETAILS OF THE INVENTION

The resulting fluorinated alkane produced in accordance with the invention has one or more fluorine atoms over and above the number of fluorine atoms originally present in the halogenated alkane.

The halogenated alkane starting materials of the invention do not substantially react with hydrogen fluoride alone under the conditions of temperature and pressure used in this invention and require the presence of added catalyst, specifically tantalum pentachloride (TaCl₅) or tantalum pentabromide (TaBr₅). It is preferred that the TaCl₅ or TaBr₅ be used in an amount from 0.001 to 5 moles, more preferably 0.001 to 0.250 mole per mole of starting halogenated alkanes for reasons of economy and effectiveness. TaCl₅ is the preferred catalyst. The catalyst is a commercially available crystalline solid and can be used alone or on a support, such as carbon.

The preferred halogenated alkanes of the formula R¹R²R³R⁴C are wherein x = 0 to 3, and more preferably from 0 to 1. n is preferably from 4 to 6. In a particularly preferred embodiment R1 = CₓZ₂ₓ+1, wherein x = 1 and R², R³, and R⁴ are selected from H, F, Cl and Br. In addition to the proviso that at least one of R¹, R², R³ and R⁴ be Cl or Br, or contain Cl or Br. It is also preferred that at least one of R¹, R², R³ and R⁴ be H. The specifically preferred halogenated alkanes are selected from CHCl₃, CH₂Cl₂, CHCl₂CHCl₂, CH₂ClCCl₃, CCl₂FCH₂Cl, CClF₂CH₂Cl, CF₃CHCl₂, CHClF₂ and CHCl₂F.

The reaction can be carried out in the liquid phase or vapor phase and at autogenous pressures or under constant pressure ranging from atmospheric to superatmospheric. Both the liquid phase and vapor phase processes include batch, semicontinuous and continuous modes of operation.

The reaction is carried out at from O°C to 185°C. The preferred temperature is 35°C to 175°C.

Anhydrous or substantially anhydrous conditions means that water, which is detrimental to the reaction, should be excluded as much as possible from the reaction zone. The HF which is commercially available as anhydrous grade can be used in the reaction directly. It is preferred that 1 to 30 molar equivalents of HF be utilized. Exclusion of moisture from the reaction vessel by means of appropriate moisture traps or other means is a routine procedure and is well known in the art.

The reaction vessel is constructed from materials which are resistant to the action of hydrogen halide such as nickel alloys including monel, "Hastelloy" and "Inconel".

The liquid phase reactions are conducted by introducing the reagents in any order into the reaction vessel. Generally, the TaCl₅ or TaBr₅ and starting halogenated alkane are placed in the reaction vessel which is then cooled, and the required amount of hydrogen fluoride is condensed in the vessel. The vessel may be evacuated prior to the introduction of hydrogen fluoride and cooled in Dry Ice or liquid nitrogen to facilitate addition of the hydrogen fluoride. The contents of the vessel are raised to the appropriate reaction temperature and agitated by shaking or stirring for a length of time sufficient to cause the reaction to occur.

For liquid phase reactions, the amount of TaCl₅ or TaBr₅ used is from 0.001 to 5 moles, preferably 0.001 to one mole, per mole of starting halogenated alkane, and more preferably from 0.001 to 0.250 mole and in some cases from 0.005 to 0.1 mole per mole of starting halogenated alkane. The amount of HF used in the reaction is from 1 to 30 molar equivalents per mole of halogenated alkane. The reaction can be carried out at from O°C to 185°C. The preferred temperature is 35°C to 175°C. Reaction time can be from 0.5 to 18 hours; the preferred times are from 1 to 8 hours.

In the vapor phase reaction, the reactants are introduced into the reactor above their boiling points. The temperature of the reactor must also be sufficient to keep the products of the reaction in the vapor state so that they pass over into a cooled receiver beyond the reactor rather than remain in the catalyst zone for a prolonged period of time.

For vapor phase reactions, it is convenient to support the catalyst on an inert porous material such as carbon or other known supports. The amount of TaCl₅ or TaBr₅ to inert support is from 10% to 50% by weight with amounts of about 25% being preferred. The amount of HF used in the reaction is from 1 to 30 molar equivalents per mole of starting halogenated alkane. The reaction can be carried out at from 50°C to 185°C. The preferred temperature is 70°C to 170°C. The contact time of the reagents with the catalyst may be specified instead of reaction time. The combined operations of feed rate, control of reactor temperature and pressure and rate of removal of product from the reactor influence the residence time of the product in the reactor. It may be desirable to shorten the residence time for a given product within the reactor to control the formation of undesired products. Contact time is the average time that the reactant product mixture is in contact with the catalyst. Broadly, contact times of from 0.1 to 25 seconds are useful with preferred contact times in the range of 1 to 10 seconds.

Under the reaction conditions set forth above, a portion of the TaCl₅ or TaBr₅ may undergo fluorination, so that a portion of the TaCl₅ or TaBr₅ may be in the form of TaCl₅₋ₓFₓ or TaBr₅₋ₓFx. The instant invention is understood to include that condition where it may exist.

Pressure is not critical. Atmospheric, superatmospheric and autogeneous pressures are the most convenient and are therefore preferred.

The fluorinated alkanes produced by the invention have utility as refrigerants, solvents and blowing agents.

### EXAMPLES

### General Experimental Procedure

The reactor consisted of a 100 ml high pressure cylinder made of monel or "Inconel" containing a magnetic stirrer and an internal thermocouple. Mounted on top of the reactor was a condenser and a back pressure regulator connected to an optional on line analytical system. Suitable inlet and exit lines were also present to allow for admission of reactants and withdrawal of products.

To the reactor was charged the TaCl₅ in the desired amount. The reactor was then cooled and evacuated. The halogenated alkane starting material and the required amount of HF was then admitted to the reactor. It was then pressurized with nitrogen to the desired pressure while still cold and then gradually brought to the desired operating temperature with stirring by using external heat provided with an oil bath. The back pressure regulator was set to the desired operating pressure prior to heating the reactor.

At the completion of the reaction, the product was isolated by conventional means and analyzed by gas chromatography. All the percentages reported in the Examples are area percent.

### EXAMPLE 1

The General Experimental Procedure was followed using 20.25 g of pentachloroethane, 4.0 g of tantalum pentachloride and 15 g of anhydrous HF. The reactor was pressurized to 13.6 bar (200 psig) with nitrogen when cold and the back pressure regulator was set for 34 bar (500 psig). The contents were heated with stirring at an internal temperature of 142-144°C for about one hour. Analysis indicated 76.9% CF₃CHCl₂ and 21.3 CClF₂CHCl₂ as the major products.

### EXAMPLE 2

The General Experimental Procedure was followed using 16.8 g of 1,1,1,2-tetrachloroethane, 4.0 g of tantalum pentachloride and 6 g of anhydrous HF. The reactor was pressurized to 13.6 bar (200 psig) with nitrogen when cold and the back pressure regulator was set for 34 bar (500 psig). The contents were heated with stirring at 75-80°C for 45 minutes. Analysis indicated 16.6% CF₃CH₂Cl and 72.3% CClF₂CH₂Cl as the major products.

### EXAMPLE 3

Example 2 was repeated with the exception that 0.5 9 of tantalum pentachloride was used and the back pressure regulator was set for 17 bar (250 psig). The contents were heated with stirring at 60-65°C for 90 minutes. Analysis indicated 4.6% CF₃CH₂Cl, 75.1% CClF₂CH₂Cl, 15.7% CCl₂FCH₂Cl and 3.9% unreacted starting material in addition to small amounts of other organics.

### EXAMPLE 4

The General Experimental Procedure was followed using 34 g of 1,1,1-trichloroethane, 0.2 g of tantalum pentachloride and 5 g of anhydrous HF. The reactor was pressurized to 3.4 bar (50 psig) with nitrogen when cold and the back pressure regulator was set at 6.8 bar (100 psig). The contents were heated with stirring at 36-38°C for about two hours. Analysis indicated 7.9% CH₃CClF₂ 32.5% CH₃CCl₂F and unreacted starting material.

### EXAMPLE 5

The General Experimental Procedure was followed using 20.4 g of CClF₂CCl₃, 3.0 g of tantalum pentachloride and 10 g of anhydrous HF. The reactor was pressurized to 13.6 bar (200 psig) with nitrogen when cold and the back pressure regulator was set for 27.2 bar (400 psig). The contents were heated with stirring at 120-130°C for two hours. Product analysis indicated 96.4% CClF₂CCl₂F and 2.5% unreacted starting material and small amounts of other organics.

### EXAMPLE 6

Example 5 was repeated except that CCl₂FCCl₂F was used as the starting material. Product analysis indicated 80.6% CClF₂CCl₂F and 18.6% starting material in addition to small amounts of other organics.

### EXAMPLE 7

The General Experimental Procedure was followed using 18.6 g of CF₃CCl₃, 3.0 g of tantalum pentachloride and 10 g anhydrous HF. The reactor was pressurized to 13.6 bar (200 psig) with nitrogen when cold and the back pressure regulator was set for 27.2 bar (400 psig). The contents were heated with stirring at 128-133°C for about two hours. Product analysis indicated 37% CF₃CCl₂F in addition to essentially unreacted starting material.

### EXAMPLE 8

The General Experimental Procedure was followed using 30.8 g of carbon tetrachloride, 3.0 g of tantalum pentachloride and 10 9 of anhydrous HF. The reactor was pressurized to 13.6 bar (200 psig) with nitrogen when cold and the back pressure regulator was set for 34 bar (500 psig). The contents were heated with stirring at 53-57°C for about one hour. At the end of this period, off gas analysis indicated >96% CCl₂F₂. The reactor pressure was carefully vented to remove CCl₂F₂. The organic product from the reactor after the CCl₂F₂ was vented weighed 4.2 g. Analysis of this product showed 1.1% CCl₂F₂, 96.6% CCl₃F and 2.8% carbon tetrachloride in addition to small amounts of other organics.

### EXAMPLE 9

Example 8 was substantially repeated except 23.6 g of chloroform was used as the starting halogenated alkane. The contents were stirred and heated at 63-65°C for about one hour. Off gas analysis indicated >95% CHClF₂. The reactor pressure was vented to remove most of the CHClF₂ and the liquid organic product remaining in the reactor (7.5 g) was analyzed and found to contain 10.3% CHClF₂, 40.8% CHCl₂F and 46.9% chloroform in addition to small amounts of other organics.

## Claims

1. A process for the preparation of fluorinated alkanes by contacting at a temperature of O°C to 185°C under substantially anhydrous conditions, one molar equivalent of a starting material selected from halogenated alkanes of the following formulas
R¹R²R³R⁴C and R⁵R⁶R⁷R⁸C
wherein
R¹, R², R³, R⁴, are CₓZ₂ₓ+1, wherein Z is H, F, Br, or Cl and x = 0 to 10 with the proviso that at least one of R¹, R², R³, or R⁴ be Cl or Br or contain Cl or Br;
R⁵ and R⁶ taken together are -(CH₂)ₙ wherein n is an integer from 2 to 7, wherein R⁷ and R⁸ are CₓZ₂ₓ+1, wherein Z is H, F, Br, or Cl and x = 0 to 10, with the proviso that at least R⁷ or R⁸ is Cl or Br or contains Cl or Br, with HF in the presence of at least one catalyst selected from tantalum pentachloride and tantalum pentabromide to produce reaction products; removing the reaction products from contact with the catalyst and isolating a fluorinated alkane having one or more fluorine atoms above the number present in the starting material.

2. The process of Claim 1 wherein the amount of HF is 1 to 30 molar equivalents.

3. The process of Claim 1 wherein the catalyst is present in an amount of 0.001 to 0.250 molar equivalent.

4. The process of Claim 1 wherein the catalyst is tantalum pentachloride.

5. The process of Claim 1 wherein the temperature is 35°C to 175°C.

6. The process of Claim 1 wherein at least one of R¹. R², R³ or R⁴ is H.

7. The process of Claim 1 wherein the halogenated alkane is selected from CHCl₃, CH₂Cl₂, CHCl₂CHCl₂, CH₂ClCCl₃, CCl₂FCH₂Cl, CClF₂CH₂Cl, CF₃CHCl₂, CHClF₂ and CHCl₂F.

8. The process of Claim 1 wherein the catalyst is present in an amount of 0.001 to 5 molar equivalents.

## Patentansprüche

1. Verfahren zur Herstellung fluorierter Alkane durch Zusammenbringen bei einer Temperatur von 0 °C bis 185 °C unter im wesentlichen wasserfreien Bedingungen von einem Moläquivalent eines Ausgangsmaterials, ausgewählt aus halogenierten Alkanen der folgenden Formeln:
R¹R²R³R⁴C und R⁵R⁶R⁷R⁸C
worin R¹, R², R³, R⁴, CₓZ₂ₓ+1 bedeutet, worin Z für H, F, Br oder Cl steht und x eine Zahl von 0 bis 10 darstellt, mit der Maßgabe, daß wenigstens eines von R¹, R², R³ oder R⁴ Cl oder Br darstellt oder Cl oder Br enthält;
R⁵ und R⁶ zusammengenommen -(CH₂)ₙ bedeuten, worin n für eine ganze Zahl von 2 bis 7 steht, worin R⁷ und R⁸ CₓZ₂ₓ+1 bedeuten, worin Z H, F, Br oder Cl darstellt, und X = 0 bis 10, mit der Maßgabe, daß wenigstens R⁷ oder R⁸ Cl oder Br bedeutet oder Cl oder Br enthält, mit HF in Gegenwart eines Katalysators, ausgewählt aus Tantalpentachlorid-und Tantalpentrabromid, um Reaktionsprodukte herzustellen, Entfernen der Reaktionsprodukte von dem Kontakt mit dem Katalysator und Isolieren eines fluorierten Alkans mit einem oder mehreren Fluoratomen, die in der Anzahl diejenigen übersteigen, die in dem Ausgangsmaterial vorhanden sind.

2. Verfahren nach Anspruch 1, bei die Menge an HF 1 bis 30 Moläquivalente beträgt.

3. Verfahren nach Anspruch 1, bei dem der Katalysator in einer Menge von 0,001 bis 0,250 Moläquivalenten vorhanden ist.

4. Verfahren nach Anspruch 1, bei dem der Katalysator Tantalpentachlorid ist.

5. Verfahren nach Anspruch 1, bei dem die Temperatur 35 °C bis 175 °C beträgt.

6. Verfahren nach Anspruch 1, bei dem wenigstens eines von R¹, R², R³ oder R⁴ für H steht.

7. Verfahren nach Anspruch 1, bei dem das halogenierte Alkan ausgewählt wird aus CHCl₃, CH₂Cl₂, CHCl₂CHCl₂, CH₂ClCCl₃, CCl₂FCH₂Cl, CClF₂CH₂Cl, CF₃CHCl₂, CHClF₂ und CHCl₂F.

8. Verfahren nach Anspruch 1, bei dem der Katalysator in einer Menge von 0,001 bis 5 Moläquivalenten vorhanden ist.

## Revendications

1. Procédé de préparation d'alcanes fluorés par mise en contact, à une température de 0°C à 185°C, dans des conditions substantiellement anhydres, d'un équivalent molaire d'une matière de départ choisie parmi les alcanes halogénés représentées par les formules suivantes:
R¹R²R³R⁴C et R⁵R⁶R⁷R⁸C
dans lesquelles
R¹, R², R³, R⁴ sont CₓZ₂ₓ₊₁, dans laquelle Z est H, F, Br ou Cl et x = 0 à 10, avec la condition qu'au moins l'un des radicaux R¹, R², R³ ou R⁴ est Cl ou Br ou contient Cl ou Br;
R⁵ et R⁶ pris ensemble sont -(CH₂)ₙ, dans laquelle n est un nombre entier de 2 à 7, R⁷ et R⁸ sont CₓZ₂ₓ₊₁, dans laquelle Z est H, F, Br ou Cl et x = 0 à 10, avec la condition qu'au moins R⁷ ou R⁸ est Cl ou Br ou contient Cl ou Br, et de HF en présence d'au moins un catalyseur choisi parmi le pentachlorure de tantale et le pentabromure de tantale pour générer les produits de la réaction; la séparation des produits de la réaction d'avec le catalyseur et l'isolement d'un alcane fluoré ayant un ou plusieurs atomes de fluor de plus que le nombre présent dans la matière de départ.

2. Procédé selon la Revendication 1, dans lequel la quantité de HF est de 1 à 30 équivalents molaires.

3. Procédé selon la Revendication 1, dans lequel le catalyseur est présent dans une quantité de 0,001 à 0,250 équivalent en moles.

4. Procédé selon la revendication 1, dans lequel le catalyseur est le pentachlorure de tantale.

5. Procédé selon la Revendication 1, dans lequel la température est de 35°C à 175°C.

6. Procédé selon la Revendication 1, dans lequel au moins un des radicaux R¹, R², R³ ou R⁴ est H.

7. Procédé selon la Revendication 1, dans lequel l'alcane halogéné est choisi parmi CHCl₃, CH₂Cl₂, CHCl₂CHCl₂, CH₂ClCCl₃, CCl₂FCH₂Cl, CClF₂CH₂Cl, CF₃CHCl₂, CHClF₂ et CHCl₂F.

8. Procédé selon la Revendication 1, dans lequel le catalyseur est présent dans une quantité de 0,001 à 5 équivalents molaires.
